# FASCICULE DE BREVET EUROPEEN

(11) **EP 1 297 176 B1**
(45) Date de publication et mention de la délivrance du brevet: **19.07.2006**
(21) Numéro de dépôt: 01951775.4
(22) Date de dépôt: 04.07.2001
(51) Int. Cl.: C12Q 1/02, C12R 1/225

(54) **MICROORGANISMES AYANT UNE ACTION MODULATRICE DE LA GLYCOSYLATION DE SURFACE DES CELLULES INTESTINALES**
MIKROORGANISMEN, DIE AUF DIE OBERFLÄCHENGLYKOLYSIERUNG VON INTESTINALEN ZELLEN EINWIRKEN
MICRO-ORGANISMS WITH GLYCOSYLATION MODULATING ACTION OF INTESTINAL CELL SURFACE

(30) Priorité: 04.07.2000 FR 0008672
(43) Date de publication de la demande: 02.04.2003
(73) Titulaire: COMPAGNIE GERVAIS DANONE, 92300 Levallois Perret (FR)
(72) Inventeur: ANTOINE, Jean-Michel, F-94700 Maisons-alfort (FR); FREITAS, Miguel, F-75003 Paris (FR); CAYUELA, Chantal, F-75016 Paris (FR); TRUGNAN, Germain, F-93100 Montreuil (FR)
(74) Mandataire: Vercaemer, Laurence
(86) Numéro de dépôt international: PCT/FR2001/002147
(87) Numéro de publication internationale: WO 2002/002800

(56) Documents cités:
- WO-A-99/29833
- MACK D R ET AL: "Modulation in the expression of intestinal MUC3 mucin by non-pathogenic probiotic microbes." DIGESTIVE AND LIVER DISEASE, vol. 32, no. Supplement 1, mai 2000 (2000-05), page A29 XP001001545 International Meeting on Inflammatory Bowel Diseases;Capri, Italy; June 18-21, 2000
- BRY LYNN ET AL: "A model of host-microbial interactions in an open mammalian ecosystem." SCIENCE (WASHINGTON D C), vol. 273, no. 5280, 1996, pages 1380-1383, XP000993528 ISSN: 0036-8075 cité dans la demande
- HOOPER LORA V ET AL: "Host-microbial symbiosis in the mammalian intestine: Exploring an internal ecosystem." BIOESSAYS, vol. 20, no. 4, avril 1998 (1998-04), pages 336-343, XP001003006 ISSN: 0265-9247

## Description

L'invention a pour objet les microorganismes ayant un effet modulateur de la glycosylation de surface ou de la composition en sucres à la surface des cellules intestinales, ainsi qu'une méthode de sélection desdits microorganismes, et leurs utilisations dans les domaines alimentaire et médical.

La muqueuse gastro-intestinale consiste en une couche simple de cellules épithéliales recouverte au moins partiellement du côté de la lumière intestinale d'une couche visco-élastique constituée principalement de glycoconjugués. Les cellules épithéliales synthétisent les glycoconjugés présents à leur surface, qui sont les intermédiaires de nombreuses interactions, en particulier avec des lectines ou adhésines, avec des toxines bactériennes, ou avec des anticorps, des bactéries, des virus, des parasites. Ces glycoconjugués constituent donc des intermédiaires importants dans les relations entre l'hôte et la flore intestinale.

Ces glycoconjugués sont, comme leur nom l'indique, des composés glycosylés, c'est-à-dire sur lesquels sont greffées des chaînes de sucres plus ou moins longues et éventuellement ramifiées. Dans l'intestin humain sain, ces sucres peuvent être le galactose (Gal), le fucose (Fuc), l'acide N-acétylneuraminique ou acide sialique, la N-acétylglucosamine (GlcNAc), la N-acétylgalactosamine (GalNac), reliés entre eux par différentes liaisons. Trois d'entre eux peuvent être en position terminale ; il s'agit du galactose, du fucose et de l'acide sialique.

Un hôte et sa microflore intestinale fonctionnent comme un système complexe dans lequel la microflore a un impact significatif sur l'hôte. Dans le cas de microorganismes non-pathogènes ou probiotiques, on se trouve souvent en présence d'une relation symbiotique ou de coopération entre l'hôte et les microorganismes, la présence de ces derniers étant nécessaire au bon équilibre et au bon fonctionnement de l'intestin de l'hôte. Au contraire, la présence de microorganismes pathogènes, plus rare, peut avoir des conséquences négatives, en empêchant ou diminuant la présence des microorganismes probiotiques, voire même en ayant une action de parasitisme directement néfaste pour l'hôte.

L'équilibre fragile entre l'hôte et la microflore est directement lié à l'environnement intestinal et en particulier à la présence quantitative et qualitative des glycoconjugués de surface. En effet, on sait que certains microorganismes, probiotiques ou pathogènes, seront sensibles à certains sucres en position terminale. Les bactéries intestinales peuvent moduler le schéma de glycosylation des glycoconjugués présents à la surface des cellules intestinales, sans que leur mode d'action soit complètement éclairci comme on le verra ci-après. Les bactéries peuvent, d'une part, induire la présence de tel ou tel sucre lors de la glycosylation et, d'autre part, dégrader les sucres présents en position terminale d'une chaîne, ce qui modifie qualitativement et/ou quantitativement les sucres présents en position terminale.

Cette modulation du schéma de glycosylation de surface des cellules épithéliales entraîne une modification de l'environnement intestinal, l'environnement modifié pouvant favoriser l'implantation de certains microorganismes et/ou limiter, voire éviter, l'implantation d'autres microorganismes. Une modification de l'environnement, éventuellement généré par la microflore, a donc un impact direct sur l'équilibre entre l'hôte et la microflore.

Il serait donc très intéressant de disposer d'un modèle permettant de sélectionner rapidement et facilement des microorganismes en fonction de leur action sur le schéma de glycosylation. On pourrait ainsi identifier des microorganismes favorisant la présence de tel ou tel sucre, et donc favorisant l'implantation de microorganismes bénéfiques comme par exemple des probiotiques et/ou limitant l'implantation de microorganismes pathogènes.

En effet, d'après les connaissances de la Demanderesse, on n'a pas à ce jour identifié de microorganismes, en particulier de bactéries lactiques, capables d'agir finement, avec précision, sur le schéma de glycosylation, et ainsi de moduler avec précision ce schéma de glycosylation et donc l'implantation de microorganismes.

De tels microorganismes modulateurs du schéma de glycosylation pourraient trouver une utilisation en particulier dans des compositions pharmaceutiques ou alimentaires ou compléments alimentaires. En effet, ils peuvent permettre d'optimiser le fonctionnement des cellules intestinales et le bon équilibre de la microflore.

BioEssays, 1998, 20:336-343, faisant entre autres référence à Science, 1996, 273:1380-1383, décrit des méthodes dans lesquelles les microorganismes sont incubées in vivo et dans lesquelles l'expression de gènes est analysée.

Ces documents, ainsi que le document Digestive and Liver Disease, 2000, 32(S1):A29, abrégé N°: 100), décrivent des souches de bactéries lactiques qui induisent une modulation de la glycosylation de surfaces des cellules épithéliales intestinales.

Le document WO-A-99 29833 décrit l'utilisation d'une souche de bactéries lactiques pour la préparation d'une composition alimentaire.

Le document WO 99/29833 décrit une nouvelle souche de bactérie présentant certaines propriétés, par exemple activité anti-microbienne. Cependant, les modalités de son action ne sont pas détaillées, et en particulier la question de l'éventuelle action de la bactérie sur les sucres n'est jamais abordée.

Bry L. et al., dans « A model of host-microbial interactions in an open mammalian ecosystem », Science, Vol. 273, pp. 1380-1383, Sept. 6, 1996, ont étudié in vivo (chez la souris) l'influence de *Bacteroides thetaiotaomicron* sur la fucosylation. *Bacteroides thetaiotaomicron* fait partie de la flore intestinale chez l'homme et chez la souris. Le modèle utilisé est cependant limité à l'étude de la fucosylation, à l'exclusion des autres types de glycosylation. C'est d'autre part un modèle in vivo, donc plus long et complexe à mettre en oeuvre qu'un modèle in vitro.

Toutefois, ce modèle a permis d'émettre l'hypothèse de l'existence d'un facteur soluble sécrété par *B. thetaiotaomicron*, et qui jouerait le rôle d'un signal entraînant la modification de la glycosylation de surface. Ce facteur soluble, non identifié, agirait donc sans qu'il y ait contact direct entre les bactéries et les cellules cibles.

A la connaissance de la Société Demanderesse, aucune méthode n'a été développée à ce jour permettant de sélectionner in vitro, de manière facile et rapide, différents microorganismes, en particulier différentes souches de bactéries lactiques, en fonction de leur action précise sur le schéma de glycosylation des cellules épithéliales intestinales, et plus précisément sur la modulation ou variation de la composition en chacun des sucres.

Dans le cadre de la présente demande, le terme « bactéries lactiques » désigne les bactéries susceptibles de produire de l'acide lactique, et notamment les bactéries non pathogènes choisis dans le groupe comprenant *Streptococcus, Lactobacillus, Lactococcus, Bifidobacterium et Leuconostoc, en particulier B. breve, B. longum, L. lactis, S. thermophilus, L. casei, L. helveticus et L. bulgaricus*.

Un objet de la présente invention est donc une telle méthode de sélection in vitro, qui comprend les étapes suivantes :
- mise en contact du surnageant de culture de la souche de microorganismes, en particulier de bactéries lactiques, avec des cellules d'un modèle de lignée cellulaire représentant les cellules épithéliales intestinales,
- incubation,
- extraction des cellules et incubation avec différentes lectines couplées à un fluorochrome,
- lavage,
- mesure de l'intensité de fluorescence moyenne (IFM) de chaque lectine, comparaison avec les mesures effectuées sur un échantillon témoin pour évaluer la variation d'IFM induite par les bactéries, sélection de la souche de microorganismes induisant une variation d'au moins 20% pour au moins un sucre.

Cette dernière étape peut notamment être réalisée sur plaques, la mesure de l'IFM étant mesurée par sonde, selon des techniques connues de l'homme du métier, ou par cytométrie de flux. Dans le cas de la cytométrie de flux, on peut aussi effectuer la comparaison du graphe obtenu, représentant l'évolution de la fluorescence en fonction du nombre de cellules avec celui obtenu avec un échantillon témoin, et sélectionner la souche de microorganismes induisant une variation de l'allure du graphe.

On utilise dans cette méthode de sélection un modèle quelconque de lignée cellulaire représentant les cellules épithéliales intestinales. L'homme de l'art saura choisir un modèle de lignée approprié parmi les modèles connus, par exemple les lignées Caco-2 ou HT29-MTX, qui sont des cellules épithéliales intestinales cancéreuses en culture. Ces lignées sont considérées comme reproduisant tout ou partie des mécanismes de régulation des systèmes in vivo et en particulier la production de glycoconjugués. Toute lignée présentant cette caractéristique peut être utilisée dans la méthode selon l'invention. Concernant la lignée HT29-MTX, on pourra se référer à Lesuffleur et al., *« Growth adaptation to methotrexate of HT-29 human colon carcinoma cells is associated with their ability to differentiate into columnar absorptive and mucus-secreting cells* », 1990, Cancer Res., 50:6334-6343.

On effectue une centrifugation et une filtration du milieu de culture dans lequel a été incubée chaque souche de microorganismes à tester, afin d'en extraire le surnageant (« essai »). Sans vouloir être liée par une quelconque théorie, la Demanderesse estime que le surnageant contient le facteur soluble constituant le signal de modulation de la glycosylation. Le surnageant constitue donc une fraction active de souche de microorganismes. Mis en contact avec les cellules épithéliales intestinales, il va entraîner ou non telle ou telle modification de la glycosylation en fonction de la souche de microorganismes testée.

Ainsi chez *Bacteroides,* le facteur soluble a été identifié comme étant une molécule de faible poids moléculaire (inférieur à 8 kD) et qu'il est sensible à la chaleur. On peut supposer que le facteur soluble d'une autre souche présentera des caractéristiques similaires sinon identiques.

Le surnageant obtenu à partir du milieu de culture qui n'a pas été en contact avec les bactéries est utilisé comme contrôle (« témoin »).

On incorpore une quantité appropriée des surnageants « essai » et « témoin » au milieu de culture dans lequel sont placées les cellules du modèle de lignée cellulaire, par exemple HT29-MTX, pendant une période appropriée. Cette période peut être, par exemple, de 1 à 15 jours.

Dans un mode de réalisation particulier, on peut ajouter, à la place du surnageant, des bactéries. Dans ce cas, on pourra ajouter les bactéries sous forme de suspension en présence de pénicilline G et d'un tampon fort permettant d'inhiber le développement bactérien sans empêcher la viabilité des bactéries, et acidification du milieu.

Après cette période d'incubation, les cellules du modèle de lignée cellulaire sont détachées et resuspendues, et le même nombre de cellules « essai » et « témoin » est incubé séparément avec une lectine dont on connaît la spécificité vis à vis des sucres (nature du sucre, nature de la liaison).

Chaque lectine, qui est couplée à un fluorochrome, va se fixer sur le sucre dont elle est spécifique.

Les suspensions cellulaires « essai » et « témoin » sont chacune lavées plusieurs fois, et la réactivité de chaque lectine est quantifiée par mesure de la fluorescence appelée IFM (intensité de fluorescence moyenne).

Cette mesure peut être effectuée par tout moyen approprié, en particulier au moyen de la cytométrie de flux (FACScan , Bencton-Dickinson) ou par mesures fluorimétriques directes sur plaques.

Lorsqu'on utilise la cytométrie de flux, on obtient pour chaque lectine un graphe représentant l'évolution de la fluorescence en fonction du nombre de cellules. Pour chaque graphe de cytométrie de flux, l'intensité de fluorescence moyenne correspond à la valeur médiane.

Deux comparaisons sont possibles. D'une part, quel que soit le moyen de mesure de l'intensité de fluorescence, on peut comparer quantitativement l'IFM « essai » avec l'IFM « témoin ». D'une part, dans le cas de la cytométrie de flux, on peut également comparer qualitativement le résultat « essai » avec le résultat « témoin », et plus précisément comparer le graphe obtenu avec la suspension « essai » avec celui obtenu avec la suspension « témoin », c'est-à-dire comparer l'allure des courbes.

On estime qu'une diminution ou une augmentation de cette IFM d'au moins environ 20%, de préférence au moins environ 28%, plus préférentiellement encore d'au moins environ 35%, révèle un changement significatif de la réactivité de chaque lectine, donc de la composition en sucres des glycoprotéines présentes à la surface des cellules épithéliales.

Cette méthode permet donc d'observer et de quantifier de manière simple et rapide la présence de chaque lectine sur les chaînes de sucre, donc de quantifier le sucre dont la lectine est spécifique. On peut alors comparer les valeurs obtenues sans et avec microorganismes, et ainsi évaluer les effets modulateurs de la glycosylation de chaque souche de microorganismes, afin de sélectionner les souches ayant l'effet souhaité : augmentation de tel sucre, diminution de tel autre.

L'invention a également pour objet les souches de microorganismes, en particulier de bactéries lactiques, susceptibles de moduler le schéma de glycosylation de surface des cellules épithéliales intestinales, et plus particulièrement les souches de microorganismes, en particulier de bactéries lactiques, caractérisées par le fait qu'elles induisent une variation de l'allure du graphe obtenu par cytométrie de flux et/ou de l'intensité de fluorescence moyenne d'au moins environ 25%, pour au moins un sucre choisi parmi le galactose (Gal), le fucose (Fuc), l'acide N-acétylneuraminiclue, la N-acétylglucosamine (GlcNAc), la N-acétylgalactosamine (GalNac), dans un test comprenant les étapes suivantes :
- mise en contact du surnageant de culture de la souche de bactéries avec des cellules HT29-MTX, modèle de lignée cellulaire représentant les cellules épithéliales intestinales,
- incubation,
- extraction des cellules et incubation avec différentes lectines couplées à un fluorochrome,
- lavage,
- mesure de l'intensité de fluorescence moyenne (IFM) de chaque lectine, et comparaison avec les mesures effectuées sur un échantillon témoin pour évaluer la variation d'IFM induite par les bactéries.

De tels microorganismes permettent donc de moduler la glycosylation, et donc de rétablir ou modifier l'environnement intestinal afin de favoriser l'implantation de microorganismes probiotiques et d'éviter ou limiter l'implantation de microorganismes pathogènes.

On sait par exemple que le récepteur permettant l'adhesion d' *Escherichia coli* contient du galactose et de l'acide sialique ; pour *Streptococcus pyogenes* et *Listeria monocytogenes,* il s'agit du galactose ; pour *Helicobacter pylori*, il s'agit du fucose ; et pour *Entamoeba histolytica*, il s'agit du galactose et de la N-acétyl galactosamine.

Les souches objet de l'invention peuvent être en particulier les souches suivantes : N° de collection CNCM I-2492, N° de collection CNCM 1-2493, N° de collection CNCM I-2494 (CNCM = Collection Nationale de Cultures de Micro-organismes - Institut Pasteur - 28, rue du Dr. Roux - 75724 Paris Cedex 15 - France), déposées le 20 juin 2000.

On peut utiliser de tels microorganismes, en particulier de telles bactéries lactiques, ou leur fraction active c'est-à-dire la fraction comprenant le facteur soluble, pour la préparation de compositions alimentaires ou de médicaments ou compléments alimentaires modulant la glycosylation de surface des cellules épithéliales intestinales. Ces compositions peuvent comprendre une souche unique, ou plusieurs souches, de microorganismes modulant la glycosylation.

Ainsi, on dispose de compositions permettant de maintenir ou rétablir un équilibre correct hôte - microflore intestinale, ou permettant de favoriser l'implantation de telle ou telle souche de microorganismes. Elles permettent donc d'optimiser le fonctionnement des cellules intestinales et le bon équilibre de la microflore. On peut en particulier utiliser des bactéries lactiques, et les incorporer à des produits laitiers.

Les souches testées dans les essais ci-après sont données à titre d'exemples non limitatifs.

### ESSAIS IN VITRO BACTERIES LACTIQUES

On effectue une centrifugation et une filtration du milieu de culture dans lequel ont été incubées pendant une nuit des bactéries lactiques (LAB = Lactic Acid Bacteria), afin d'en extraire le surnageant (« essai »).

Les souches de LAB utilisées sont les suivantes : N° de collection CNCM I-2492, N° de collection CNCM I-2493, N° de collection CNCM I-2494.

Le surnageant obtenu à partir du milieu de culture qui n'a pas été en contact avec les bactéries est utilisé comme contrôle (« témoin »).

On incorpore une quantité optimale pour chaque surnageant (entre 10% et 30%) « essai » et « témoin » au milieu de culture DMEM (« Dulbecco's Modified Eagle's Medium ») dans lequel est placée la lignée cellulaire HT29-MTX pendant 5 à 15 jours.

Après ce traitement, les cellules HT29-MTX sont détachées et resuspendues dans une solution PBS (« Phosphate Buffered Saline Solution »), et le même nombre de cellules « essai » et « témoin » est incubé séparément avec une lectine spécifique d'un sucre (nature du sucre, nature de la liaison) à concentration optimum pendant 30 minutes.

Les lectines commerciales utilisées et leur(s) épitope(s) sont les suivants :

| Lectine | Lectine | Epitope(s) |
|---|---|---|
| Nom | Abréviation | |
| *Artocarpus integrifolia* | Jak | Gal |
| *Ricinus communis* | RCA I | Gal |
| *Griffonia simplicifolia I B4* | GSI-B4 | Galα3Gal |
| *Helix pomatia* | HPA | GalNAc |
| *Datura stramonium* | DSA | GlcNAc |
| *Wheat germ* | WGA | GlcNAc et acide sialique |
| *Anguilla anguilla* | AAA | α-L-Fucose |
| *Ulex europaeus I* | UEA I | Fucα2Galβ |
| *Maackia amurensis* | MAA | ac. sialique α 2,3 |
| *Sambucus nigra* | SNA | ac. sialique α 2,6 |

Toutes ces lectines sont commercialisées par EY Laboratories Inc. (San Mateo, CA, USA) sauf RCA I, commercialisée par Sigma Aldrich (Saint-Quentin-Fallavier, France).

On obtient par cytométrie de flux, pour chaque souche de bactéries lactiques acides, et pour les cellules « témoin » et « essai », les valeurs d'intensité de fluorescence moyenne suivantes :

| CNCM I-2492 | | | |
|---|---|---|---|
| Lectine | "témoin" | "essai" | Evolution |
| RCA | 424 | 392 | - 7.5% |
| Jak | 422 | 431 | + 2% |
| DSA | 237 | 241 | + 1.6% |
| SNA | 57 | 48 | - 16% |
| HPA | 334 | 350 | + 4.5% |

| CNCM I-2493 | | | |
|---|---|---|---|
| Lectine | "témoin" | "essai" | Evolution |
| Jak | 535 | 392 | - 26.7% |
| SNA | 48 | 36 | - 25% |
| HPA | 354 | 379 | + 7% |
| MAA | 665 | 518 | - 22% |
| WGA | 1313 | 1376 | + 4.8% |
| GSI | 37 | 31 | - 16% |
| RCA | 317 | 247 | - 22% |
| DSA | 176 | 165 | - 6% |

| CNCM I-2494 | | | |
|---|---|---|---|
| Lectine | "témoin" | "essai" | Evolution |
| Jak | 346 | 446 | + 29% |
| SNA | 34 | 35 | + 2.9% |
| HPA | 748 | 593 | - 20.7% |
| AAA | 10 | 20 | + 100% |
| MAA | 668 | 644 | - 3.6% |
| WGA | 302 | 290 | - 4% |
| GSI | 30 | 32 | + 6.7% |
| RCA | 258 | 369 | + 43% |
| DSA | 176 | 198 | + 11% |

Ainsi, on peut immédiatement constater l'action modulatrice de la glycosylation de chaque souche de bactéries. Par exemple, on peut constater que la souche CNCM I-2494 entraîne une augmentation de Gal et de α-L-Fucose, et une diminution de GalNAc.

## Revendications

1. Méthode de sélection de souches de microorganismes comprenant les étapes suivantes :
- mise en contact du surnageant de culture de la souche de microorganismes, avec des cellules d'un modèle de lignée cellulaire représentant les cellules épithéliales intestinales,
- incubation,
- extraction des cellules et incubation avec différentes lectines couplées à un fluorochrome,
- lavage,
- mesure de l'intensité de fluorescence moyenne (IFM) de chaque lectine, comparaison avec les mesures effectuées sur un échantillon témoin pour évaluer la variation d'IFM induite, et sélection de la souche de microorganismes induisant une variation d'au moins environ 20%, pour au moins un sucre.

2. Méthode selon la revendication 1, **caractérisée en ce que** l'intensité de fluorescence moyenne est mesurée par cytométrie de flux, et correspond à la valeur médiane du graphe obtenu représentant l'évolution de la fluorescence en fonction du nombre de cellules.

3. Méthode selon l'une ou l'autre des revendications 1 et 2, **caractérisée en ce que** les microorganismes sont des bactéries, en particulier des bactéries lactiques.

4. Méthode selon l'une quelconque des revendications 1 à 3, **caractérisée en ce que** la souche de microorganismes sélectionnée induit une variation d'IF d'au moins environ 28%, de préférence au moins environ 35%.

5. Méthode selon l'une quelconque des revendications 1 à 4, **caractérisée en ce que** les cellules du modèle de lignée cellulaire sont des cellules HT29-MTX.

6. Souche de bactéries lactiques **caractérisée par le fait qu'**elle induit une variation de l'allure du graphe obtenu par cytométrie de flux et/ou de l'intensité de fluorescence moyenne d'au moins environ 25%, pour au moins un sucre choisi parmi le galactose (Gal), le fucose (Fuc), l'acide N-acétylneuraminique, la N-acétylglucosamine (GlcNAc), la N-acétylgalactosamine (GalNac), dans un test comprenant les étapes suivantes :
- mise en contact du surnageant de culture de la souche de bactéries avec des cellules HT29-MTX, modèle de lignée cellulaire représentant les cellules épithéliales intestinales,
- incubation,
- extraction des cellules et incubation avec différentes lectines couplées à un fluorochrome,
- lavage,
- mesure de l'intensité de fluorescence moyenne (IFM) de chaque lectine, et comparaison avec les mesures effectuées sur un échantillon témoin pour évaluer la variation d'IFM induite par les bactéries.

7. Souche de bactéries lactiques selon la revendication 6 déposée sous le numéro CNCM I-2493.

8. Souche de bactéries lactiques selon la revendication 6 déposée sous le numéro CNCM I-2494.

9. Utilisation d'une souche de bactéries selon l'une ou l'autre des revendications 6 à 8, ou de la fraction active d'une telle souche, pour la préparation d'une composition alimentaire ou pharmaceutique ou complément alimentaire modulant la glycosylation de surface des cellules épithéliales intestinales.

10. Composition alimentaire ou pharmaceutique, **caractérisée par le fait qu'**elle comprend au moins une souche de bactéries selon l'une ou l'autre des revendications 6 à 8 ou au moins une fraction active d'une de ces souches.

11. Composition alimentaire ou pharmaceutique selon la revendication 10, **caractérisée par le fait qu'**elle module la glycosylation de surface des cellules épithéliales intestinales.

## Claims

1. A method of selecting strains of microorganisms comprising the following steps:
- bringing the culture supernatant for the strain of microorganisms into contact with cells of a cell line model representing the intestinal epithelial cells,
- incubating,
- extracting the cells and incubating with various lectins coupled to a fluorochrome,
- washing,
- measuring the mean fluorescence intensity (MFI) of each lectin, comparing with the measurements performed on a control sample in order to evaluate the variation in MFI induced, and selecting the strain of microorganisms inducing a variation of at least 20% for at least one sugar.

2. The method as claimed in claim 1, **characterized in that** the mean fluorescence intensity is measured by flow cytometry, and corresponds to the median value of the graph obtained representing the variation of fluorescence as a function of the number of cells.

3. The method as claimed in either of claims 1 and 2, **characterized in that** the microorganisms are bacteria, in particular lactic acid bacteria.

4. The method as claimed in any one of claims 1 to 3, **characterized in that** the strain of microorganisms selected induces a variation in FI of at least about 28%, preferably at least about 35%.

5. The method as claimed in any one of claims 1 to 4, **characterized in that** the cells of the cell line model are HT29-MTX cells.

6. The strain of lactic acid bacteria, **characterized in that** it induces a variation of the shape of the graph obtained by flow cytometry and/or of the mean fluorescence intensity by at least about 25%, for at least one sugar chosen among galactose (Gal), fucose (Fuc), N-acetylneuraminic acid, N-acetylglucosamine (GlcNAc), N-acetylgalactosamine (GalNac), in a test comprising the following steps:
- bringing the culture supernatant for the strain of bacteria into contact with HT29-MTX cells, cell line model representing the intestinal epithelial cells,
- incubating,
- extracting the cells and incubating with various lectins coupled to a fluorochrome,
- washing,
- measuring the mean fluorescence intensity (MFI) of each lectin, and comparing with the measurements performed on a control sample in order to evaluate the variation in MFI induced, by the bacteria.

7. A strain of lactic acid bacteria according to Claim 6, deposited under the number CNCM I-2493.

8. A strain of lactic acid bacteria according to Claim 6, deposited under the number CNCM I-2494.

9. The use of a strain of lactic acid bacteria according to any of claims 6 to 8, or of the active fraction of such a strain, for the preparation of a food or pharmaceutical composition or a food supplement modulating intestinal epithelial cell surface glycosylation.

10. A food or pharmaceutical composition, **characterized in that** it comprises at least one bacterial strain as claimed in any one of claims 6 to 8 or at least an active fraction of one of these strains.

11. The food or pharmaceutical composition as claimed in claim 10, **characterized in that** it modulates intestinal epithelial cell surface glycosylation.

## Patentansprüche

1. Verfahren zur Selektion von Mikroorganismenstämmen, umfassend die folgenden Stufen:
- Inkontaktbringen des Kulturüberstands des Mikroorganismenstamms mit den Zellen eines Modells für eine Zellinie, das intestinale Epithelzellen repräsentiert,
- Inkubation,
- Extraktion der Zellen und Inkubation mit verschiedenen Lectinen, die an ein Fluorochrom gebunden sind,
- Waschen
- Messen der mittleren Fluoreszenzintensität (IFM = l'intensie de fluorescence moyenne) jedes Lectins, Vergleich mit den an einer Vergleichsprobe durchgeführten Messungen, um die induzierte IFM-Änderung zu bestimmen, und Selektion des Mikroorganismenstamms, der eine Änderung von wenigstens einen Zucker induziert.

2. Verfahren nach Anspruch 1, **dadurch gekennzeichnet, daß** die mittlere Fluoreszenzintensität durch Strömungscytometrie gemessen wird und dem Medianwert des erhaltenen Diagramms entspricht, der die Entwicklung der Fluoreszenz als Funktion der Zahl der Zellen darstellt.

3. Verfahren nach einem der Ansprüche und 1 und 2, **dadurch gekennzeichnet, daß** die Mikroorganismen Bakterien, insbesondere Milchsäurebakterien, sind.

4. Verfahren nach einem der Ansprüche 1 bis 3, **dadurch gekennzeichnet, daß** der selektierte Mikroorganismenstamm eine IF-Änderung von wenigstens etwa 28 %, vorzugsweise etwa 35 % induziert.

5. Verfahren nach einem der Ansprüche 1 bis 4, **dadurch gekennzeichnet, daß** die Zellen des Modells für eine Zellinie HT29-MTX-Zellen sind.

6. Milchsäurebakterien-Stamm, **dadurch gekennzeichnet, daß** er eine Änderung des durch Strömungscytometrie erhaltenen Graphs und/oder der mittleren Fluoreszenzintensität von wenigstens etwa 25 % für wenigstens einen Zucker, ausgewählt unter Galactose (Gal), Fucose (Fuc), N-Acetylneuraminsäure, N-Acetylglucosamin (GlcNAc), N-Acetylgalactosamin (GalNac) in einem Test induziert, der die folgenden Stufen umfaßt:
- Inkontaktbringen des Kulturüberstands des Bakterienstamms mit HT29-MTX-Zellen, dem Modell für eine Zellinie, das intestinale Epithelzellen repräsentiert,
- Inkubation,
- Extraktion der Zellen und Inkubation mit verschiedenen Lectinen, die an Fluorochrom gebunden sind,
- Wachen,
- Messen der mittleren Fluoreszenzintensität (IFM) jedes Lectins und Vergleich mit den an einer Vergleichsprobe durchgeführten Messungen, um die durch die Bakterien induzierte IFM-Änderung zu bestimmen.

7. Milchsäurebakterien-Stamm nach Anspruch 6, der unter der Eingangs-Nr. CNCM I-2493 hinterlegt wurde.

8. Milchsäurebakterien-Stamm nach Anspruch 6, der unter der Eingangs-Nr. CNCM I-2494 hinterlegt wurde.

9. Verwendung eines Bakterienstamms nach einem der Ansprüche 6 bis 8 oder/der aktiven Fraktion eines solchen Stamms zur Herstellung einer alimentären oder pharmazeutischen Zusammensetzung oder eines Nahrungsergänzungsmittels, die/das die Oberflächenglycosylierung von intestinalen Epithelzellen moduliert.

10. Alimentäre oder pharmazeutische Zusammensetzung, **dadurch gekennzeichnet, daß** sie wenigstens einen Bakterienstamm nach einem der Ansprüche 6 bis 8 oder wenigstens eine aktive Fraktion eines dieser Stämme umfaßt.

11. Alimentäre oder pharmazeutische Zusammensetzung nach Anspruch 10, **dadurch gekennzeichnet, daß** sie die Oberflächenglycosylierung der intestinalen Epithelzellen moduliert.
